# EUROPEAN PATENT SPECIFICATION

(11) **EP 2 877 119 B1**
(45) Date of publication and mention of the grant of the patent: **23.01.2019**
(21) Application number: 13741673.1
(22) Date of filing: 18.07.2013
(51) Int. Cl.: A61C 8/00, A61C 19/06

(54) **DENTAL IMPLANT**
ZAHNIMPLANTAT
IMPLANT DENTAIRE

(30) Priority: 20.07.2012 IT GE20120072
(43) Date of publication of application: 03.06.2015
(73) Proprietor: Bellinvia, Salvatore, 33170 Pordenone (IT); Malvagna, Carolina, 33170 Pordenone (PN) (IT); Vettori, Cristiano, 33170 Pordenone (IT); Vettori, Erica, 33170 Pordenone (IT)
(72) Inventor: VETTORI, Cristiano, 33170 Pordenone (PN) (IT)
(74) Representative: Sergio, Stefano
(86) International application number: PCT/EP2013/002140
(87) International publication number: WO 2014/012669

(56) References cited:
- WO-A1-84/02264
- WO-A1-97/22308
- WO-A1-2011/092681
- ES-A1- 2 288 437
- FR-A1- 2 796 265
- US-A1- 2013 011 814

## Description

This invention concerns a dental implant. Various types of dental implants of differing forms are already known, for instance, with prosthetic connection and cylindrical or conical body. The implants mostly used are the endoseal type that have passed a series of clinical tests carried out following specific international protocols. The material generally used for said implants is titanium, a material that is able to create with a certain facility the union between the same implant and the bone (osseointegration).

WO2011/054122 discloses a medical implantable device comprising a liquefiable element.

More precisely, by osseointegration, one means the stable and lasting union between bone and implant that is able to allow the masticatory function without any complications. If, instead, osseointegration is not achieved, one must speak of fibro-integration that may mean a total failure, in which a stable bone-implant union is not created straight away or a partial failure in which the implant works without creating any problems only for a certain period of time. Among the most widespread problems that can be verified using the known implants, there is peri-implantitis which is an inflammation of the zone surrounding the implant, for which a tissue of defence is formed (the so called granulation tissue) in case of bacterial contamination of the implant surface. This tissue, in the most serious cases, may even come to replace the bone and to provoke osteomyelitis with consequent loss of the implant in a few weeks. Implants that lose osseointegration due to infectious causes have micro flora made up of spirochetes, fusobacteria and Gram negative rods and have an etiopathogenesis tightly correlated to the specific anatomical and histological morphostructure of the same tissues involved in the integration, to the superficial morphology of the fixtures and the precision of coupling of the implant components. Such particular conditions of the implant site mean that the principal etiological factor of peri-implant diseases is really that of infection.

Microbiological and clinical research has shown that an effective control of bacterial plaque reduces the possibility of peri-implant disease just as for periodontal disease. Until today, studies to overcome such problems have been concentrated on the external surface of implants using particular coatings able to contrast, at least partly, the occurrence of precocious and delayed bacterial infections, but still today it is necessary to place a pharmacological therapy in both a general and local way alongside the surgical therapy.

The subject of this invention is defined in the claims and is the realization of an implant made according to the known criteria, but that inside of which there is an empty space, of varying dimensions and shape, also identifiable as a prolongation of the receptive housing of the fixing screw or fixation of the superstructures or in a more apical or crown seating, created for this purpose, definable as a reservoir or housing, that communicates through one or more holes of various diameter with the external surface of the same implant in correspondence of any area of the smooth or rough surface (apical part, crown part, threaded part or any portion whatsoever in contact with the bone or mucous surfaces or of interface with the superstructure).

The communication of the aforesaid reservoir with the peri-implant environment can also be made by capillarity through the inside threading of the implant corresponding to the seat of insertion of the superstructure screw, with possible capillary diffusion of the substance directly inserted in the zone of implant emergency. Inside the aforesaid reservoir, one places a "tablet" made up of a disinfectant/antiseptic and/or an anti-inflammatory and/or an antibiotic/chemotherapy medicament and/or a factor stimulating bone regeneration and/or any medicine or derivate (for instance silver salts) suitable to encourage osseointegration and to contrast precocious and delayed infection. Such substance is coated by a casing that determines a controlled release of it. This invention is therefore an implant able to resolve in a meaningful way the problems listed above through prevention and treatment of infections, through release of medicine from the interior of the same implant. In such way, the release of a disinfectant and/or antimicrobial is able to contrast bacterial contamination and an inflammatory reaction. Furthermore, a growth factor may be used to accelerate the process of osseointegration.

The invention is further clarified through illustration of the exemplary solution in the attached drawings, in which:
Figure 1 illustrates a side view of the implant subject of this invention according to a first exemplary solution;
Figure 2 illustrates a central cross-section of the implant in Figure 1;
Figure 3 illustrates a side view of the implant subject of this invention according to a further exemplary solution;
Figure 4 illustrates a central cross-section of the implant in Figure 3.

The dental implant in this invention includes a threaded shaft having external threads 1 endowed with a central passage 2, with an internal portion 3, also threaded, a fixing screw 5, complementary to said internal threaded portion, including a truncated-conical/cylindrical section adapted to receive a dental crown or an attachment 6 on it.

In the terminal part of the internally threaded portion there is a housing 7, of any shape (for instance inverted truncated-conical, cylindrical or spherical), that is connected with the external part of the same implant in contact with the bone, where infectious breeding grounds can be formed, through one or more connection canaliculi 8.

Inside the housing 7, a medicinal product is inserted, for instance, in the form of a tablet 9, which may be a disinfectant or an anti-inflammatory or an antibiotic/chemotherapy medicament and even a product stimulating bone regeneration or any medicine or derivate suitable to encourage osseointegration and to contrast precocious or delayed peri-implant infection. Said tablet may be coated with a casing in such way as to determine a controlled release.

Said canaliculi can be of any convenient conformation in comparison to the axis of the implant so as to encourage the conduction of the medicament, where necessary.

If the tablet is totally consumed or it is necessary to replace the medicinal substance, it is possible to unscrew the fixing screw 5 and to insert the most opportune medicament and/or growth factor or any other useful product in the housing in that the latter is accessible through the aforesaid central passage 2. There are many advantages of this system:
- at the same time as the insertion of the implant we can obtain the release of a disinfectant and/or of an antimicrobial able to contrast bacterial contamination and an inflammatory reaction and/or a growth factor to accelerate the osseointegration;
- at any later moment, after having unscrewed the superstructure of the same implant, one may insert the medicament or growth factor or any useful material in the reservoir;
- the surgical procedure for insertion of the implant is not modified;
- with the presence of the "tablet" one preventively intervenes on the possible complications before the highlighting of a radiological clinical situation with the result of an easier control of an infection/inflammation through a smaller quantity of active ingredient;
- the waiting times for osseointegration are reduced, and at the same time the risks connected with an immediate load are reduced and secondary stability (stability in the middle/ long term) is encouraged;
- there is not the drawback of the quality-quantity deterioration of the active ingredient during the insertion of the implant in the bone;
- the mechanical resistance of the implant is not jeopardized;
- the external surface of the implant, whatever it may be, is unchanged;
- at an industrial level, the production methodology of the same implant is not changed in a significant way, the holes being easy to make with innumerable techniques.

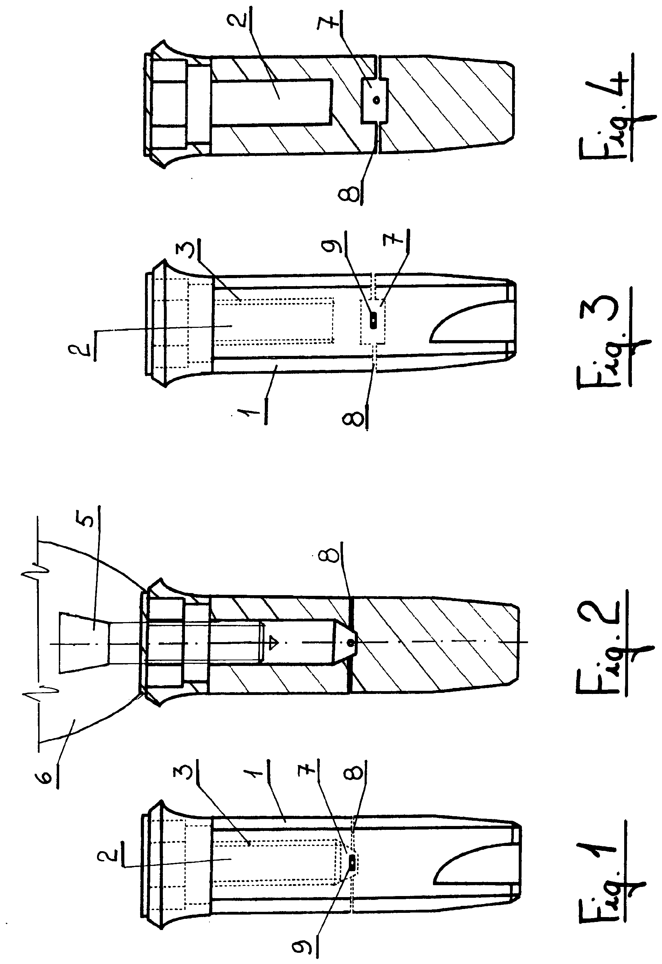

In an alternative solution illustrated in Figures 3 and 4, the housing 7 containing the medicinal tablet 9 is separated and independent from the central passage 2. In such case, the tablet containing the active ingredient is inserted inside the implant at the time of its realization or through the canaliculi of connection 8, before the insertion of the same implant in the tissue.

## Claims

1. Dental implant comprising a threaded shaft fitted with external threads (1) and a central tubular passage (2) fitted with an internal threaded portion (3), a fixing screw (5), complementary to it, which can support a dental crown or any other type of connection (6), **characterized by** the fact that, in the end portion of the central tubular passage (2), there is a housing (7) containing a tablet (9) of anti- infective and/or anti-inflammatory and/or anti-bacterial medicament and/or of a medicament stimulating tissue regeneration, or a tablet containing a substance that encourages bone growth, wherein the tablet (9) is covered by a casing so as to facilitate a controlled release of the medicament and/or of the substance encouraging bone growth.

2. Dental implant according to claim 1, wherein said housing (7) has an inverted truncated conical shape.

3. Dental implant according to claims 1 and 2, wherein said housing (7) has one or more canaliculi (8) connecting the housing (7) with the external portion of the implant itself.

4. Dental implant according to claim 3, wherein the canaliculi (8) connecting the external portion of the implant itself are longitudinal with respect to the vertical axis of the implant.

5. Dental implant according to claim 3, wherein the canaliculi (8) connecting the external portion of the implant descend from the housing (7) towards the external portion of the implant itself

## Patentansprüche

1. Zahnimplantat umfassend einen Gewindeschaft mit Außengewinde (1) und einem zentralen rohrförmigen Durchgang (2), der mit einem Innengewindeabschnitt (3) und einer hierzu komplementären Befestigungsschraube (5) versehen ist, die eine Zahnkrone oder jede andere Art von Verbindung (6) halten kann, **dadurch gekennzeichnet, dass** sich in dem Endabschnitt des zentralen rohrförmigen Durchgangs (2) ein Gehäuse (7) mit einer Tablette (9) befindet, die ein infektionshemmendes und/oder entzündungshemmendes und/oder antibakterielles Medikament und/oder ein Medikament zur Anregung der Geweberegeneration oder eine Tablette mit einer Substanz zur Anregung des Knochenwachstums enthält, wobei die Tablette (9) mit einer Hülle ummantelt ist, um eine kontrollierte Freisetzung des Medikaments und/oder der Substanz zur Anregung des Knochenwachstums zu unterstützen.

2. Zahnimplantat nach Anspruch 1, wobei das Gehäuse (7) die Form eines umgekehrten Kegelstumpfes aufweist.

3. Zahnimplantat nach den Ansprüchen 1 und 2, wobei das Gehäuse (7) einen oder mehrere Kanälchen (8) aufweist, die das Gehäuse (7) mit dem Außenabschnitt des Implantats verbinden.

4. Zahnimplantat nach Anspruch 3, wobei die den Außenabschnitt des Implantats verbindenden Kanälchen (8) in Längsrichtung in Bezug auf die vertikale Achse des Implantats verlaufen.

5. Zahnimplantat nach Anspruch 3, wobei die den Außenabschnitt des Implantats verbindenden Kanälchen (8) vom Gehäuse (7) in Richtung des Außenabschnitts

## Revendications

1. Implant dentaire comprenant un arbre fileté équipé de filets extérieurs (1) et un passage tubulaire central (2) équipé de une portion filetée intérieure (3), une vis de fixation (5), complémentaire à elle, qui peut supporter une couronne dentaire ou tout autre type de connexion (6), **caractérisé en ce que**, dans la portion d'extrémité du passage tubulaire. central (2), il y a un logement (7) contenant une pastille (9) de médicament anti-infectieux et/ou anti-inflammatoire et/ou antibactérien et/ou de un médicament stimulant la régénération de tissu, ou une pastille contenant une substance qui favorise la croissance osseuse, dans lequel la pastille (9) est recouverte par une enveloppe pour faciliter une libération contrôlée du médicament et/ou de la substance qui favorise la croissance osseuse.

2. Implant dentaire selon la revendication 1, dans lequel ledit logement (7) a une forme tronconique inversée,

3. Implant dentaire selon les revendications 1 et 2, dans lequel ledit logement (7) a un o plusieurs canalicules (8) qui connectent le logement (7) avec la portion extérieure de l'implant même.

4. Implant dentaire selon la revendication 3, dans lequel les canalicules (8) qui connectent la portion extérieure de l'implant même sont longitudinaux par rapport à l'axe vertical de l'implant.

5. Implant dentaire selon la revendication 3, dans lequel les canalicules (8) qui connectent la portion extérieure de l'implant descendent du logement (7) vers la portion extérieure de l'implant même.
